# EUROPEAN PATENT APPLICATION

(11) **EP 3 037 087 A1**
(43) Date of publication of application: **29.06.2016**
(21) Application number: 14837180.0
(22) Date of filing: 20.08.2014
(51) Int. Cl.: A61K 9/16, A61K 38/16, A61K 38/28, A61K 47/04, A61K 47/12, A61K 47/18, A61K 47/24, A61K 47/34, A61K 47/36, A61K 47/38, A61K 47/42, B01J 2/04, B01J 19/00

(54) **METHOD FOR PRODUCING MICROPARTICLES**

(30) Priority: 21.08.2013 JP 2013171688
(71) Applicant: NRL Pharma, Inc., Takatsu-ku Kawasaki-shi, Kanagawa 213-0012 (JP)
(72) Inventor: KUWATA, Hidefumi, Kawasaki-shi Kanagawa 213-0012 (JP)
(74) Representative: Appleyard Lees IP LLP
(86) International application number: PCT/JP2014/072345
(87) International publication number: WO 2015/025979

(57) **Abstract**

The present invention has an object providing microparticles having an average particle size of 100 µm or less.

The present invention provides microparticles having an average particle size of 100 µm or less and a method for producing thereof. In addition, the present invention provides medicine, food and feedstuff comprising the microparticles having an average particle size of 100 µm or less.

## Description

### TECHNICAL FIELD

The present invention relates to microparticles containing an active element(s) and having an average particle size of 100 µm or less, and to a method for producing the microparticles thereof In addition, the present invention relates to medicine, food and feedstuff comprising the microparticles containing an active element(s) and having an average particle size of 100 µm or less.

### BACKGROUND ART

A variety of medicine and food containing physiologically active components are commercially available. Some of the physiologically active components may lose their activity due to degradation or denaturation during the process of production and preservation of the product, and further after administration into a body. For example, when orally administered, a physiologically active substance such as a protein or a polypeptide is degraded and deactivated through intragastric action of the gastric acid and pepsin, and most of them lose their bioactivity. Since most of the physiologically active substances exert their functions after being absorbed from the intestinal tract or exert their action at the intestinal tract, there is a need for delivering a physiologically active component to the intestinal tract without any degradation or denaturation.

As enteric coating agents, enteric capsule materials or matrix materials for medicinal use, enteric polymers such as acrylate polymer (EUDRAGIT (registered trademark)), hydroxypropyl methylcellulose phthalate (HPMCP), cellulose acetate phthalate (CAP), hydroxypropyl methylcellulose acetate succinate (HPMCAS), and carboxy methyl ethyl cellulose (CMEC) have been used. Most of the enteric coating agents is inapplicable, since such enteric coating agents are dissolved in an organic solvent upon use and physiologically active substances such as proteins or polypeptides that are often denatued even with a tiny amount of organic solvent. Since these coating agents, enteric capsule materials and matrix materials are limited to medicinal use, naturally-derived shellac, zein or the like is used for application to food or feedstuff. Examples of use of such naturally-derived materials are disclosed, for example, as a method for protecting a protein or a polypeptide by coating it with an enteric film or by filling it in an enteric capsule (Patent Reference 1), as a matrix enteric/sustained-release composition (Patent Reference 2), as a drop-wise production method (Non-Patent References 1 and 2), and as a submerged curing method (Patent Reference 3, Non-Patent Reference 3).

These methods that employ natural sources may be applied to a tablet- or granular-type pharmaceutical product or supplements but their particle sizes are too large to be added to most of the food and pharmaceutical products having other shapes. For example, when larger particles are added to food, it may cause grainy feeling upon eating. In order to expand versatility, attempts have been made so far to produce particles with a size of a micrometer-order scale (microparticles) so that they can be added to pharmaceutical products, feedstuff and food. However, enteric coating method, sustained-release matrix preparations and submerged curing methods are difficult to produce small microparticles and there are almost no such reports. A shell material or a capsule-protecting substance is used to protect a physiologically active component into a matrix or to cover the component, but as the particle size of the particles becomes smaller, the effect of these protective components is greatly weakened. For example, when a particle with a diameter of 1 mm is compared with a particle with a diameter of 10 µm, while the 10 µm particle has a diameter that is 1/100 the diameter of the 1 mm particle, its surface area per volume, to the contrary, is greater by 100 times. Since a microparticle is not always a perfect sphere, the surface area would be even greater. For this reason, a coating agent, a matrix material or an enteric material needs to be used in large quantity for a target physiologically active component. When the surface area per volume is greater in several orders of magnitude, it is difficult to use sereral orders of magnitude of coating materials for a target component. These problems are common for both of the enteric coating agents for pharmaceutical products and the naturally-derived components.

A multi-unit tablet consisting of a matrix made of a seamless capsule and an acrylic polymer such as EUDRAGIT, a cellulose such as hydroxypropyl cellulose or methylcellulose or a fat such as hardened oil was reported (Patent Reference 4). Although this method for producing a multi-unit tablet consisting of a matrix is more versatile than a conventional method of coating tablets, the problem lies in that it can only be applied to tablets and cannot directly be applied to pharmaceutical products such as granules and liquid agents. In addition, since an acrylic polymer such as EUDRAGIT, or a cellulose such as hydroxypropyl cellulose or methylcellulose used in this method is limited to medicinal use only, there is a problem that the method also cannot be used for general food and feed. Moreover, since production takes place by releasing an active substance and a gelatin solution in a cooling oil, a multi-unit tablet as the final product inevitably contains oil, or otherwise additional steps of removing oil and washing are required.

As a sustained-release matrix preparation, a matrix preparation consisting of fish growth hormone (polypeptides with a molecular weight of about 20,000-30,000) and an enteric polymer was reported (Patent Reference 5). According to this method, particles having a size in millimeters that is smaller than tablets can be produced, but they are produced by dissolving fish growth hormone and an enteric polymer solution in ammonia water and subsequently subjecting the resultant to lyophilization, leaving problems of complicated production steps and high cost. Furthermore, pulverizing is required after the lyophilization, but even pulverized microparticles or particulate bodies having a size of a micrometer- or nano-order scale are difficult to be produced at low cost. Further problem is that in order to maintain the enteric property, the mixture ratio of the fish growth hormone and the enteric polymer required the proportion of the enteric polymer to be more than five times that of the fish growth hormone. Moreover, since the enteric polymer used is limited to medicinal use only due to the Pharmaceutical Affairs Act, it cannot be used for application to general food and feed. The ammonia water used in this production process is designated as a quasi-drug deleterious substance/hazardous substance in Japan, and its use is limited and thus cannot be used in almost all of the food and feed in other countries as well.

There is a report of a controlled-release pharmaceutical composition comprising an alginic acid gel matrix, a protein tangled to the gel matrix and a physiologically active component that can bind with the tangled protein, wherein the protein is degraded when this composition reacts under the conditions that contains a proteolytic enzyme, thereby releasing the drug (Patent Reference 6). Since the drug that can be used in this case needs to bind to the protein tangled to the gel matrix, applicable drugs are limited to inorganic compounds and organic compounds with relatively low molecular weights such as antibiotics and chemotherapeutic agents, and there is a problem that this method is inapplicable when the physiologically active component is a protein, a polypeptide or the like. The diameters of beads or pellets produced according to this method are 0.5 to 4 mm, which also raise a problem of difficulty in applying the method to microparticles or particulate bodies with a size of micrometer- to nano-order scale.

A method for producing a composition comprising a controlled delivery system, comprising the steps of: adding a target physiologically active substance and a polymer to a low-molecular-weight gelator (LMWG); and subjecting the resultant to thickening or gelation followed by drying was reported (Patent Reference 7). The gelator used in this method, however, is a chemical synthetic substance having cycloalkyl, heterocycloalkyl, an aromatic or heterocyclic aromatic moiety and an amino group in the molecular structure, which cannot be used for application to general food or feed. In addition, an additional step of drying after thickening or gelation is required, which leaves problems of complicated steps and high production cost.

There was a report of a method for producing a composition for mammal newborns, comprising the steps of: mixing a physiologically active substance with an encapsulation agent to produce a liquid composition; and subsequently drying the liquid composition (Patent Reference 8). This method can employ an encapsulation agent generally used for food, and thus is a method applicable to physiologically active substances such as proteins and polypeptides. This method, however, requires an additional step of drying after producing the liquid composition, which leaves problems of complicated steps and high production cost. In order to make capsules or particulate bodies with smaller size, the dried liquid mixture needs to be pulverized to a smaller size. Pulverization of a dried product, however, can only produce larger particles having a diameter of several-hundreds of micrometers and production of particles with a size of micrometer- to nanometer-order scale was difficult. There was also a problem of heat denaturation of the peptides or proteins during the course of pulverization for making their sizes smaller. Moreover, since the drying technique was limited to drying at a lower temperature such as lyophilization, low-temperature vacuum drying or low-temperature spray drying, there was a problem that the production process took a long period of time.

A method was reported, which comprises the steps of: dispersing a supply product containing a physiologically active substance into a spray tower in a form of droplets; simultaneously introducing a graining agent into the spray tower to coat the droplets of the sprayed supply products with the graining agent; and drying in gas at a temperature in a range of -20°C to 500°C (Patent Reference 9). Since this method owes its capsule-making mechanism to the attachment and the drying of the cloudlike graining agent on the surface of the sprayed droplets, a capsule with an average diameter of 100-2,000 µm can be produced but not a capsule with an average diameter of 100 µm or less. In addition, since the produced capsules are fragile, even though solubility of an aroma chemical or a sweetener can be delayed, it was not applicable to improve stability of the administered physiologically active substance in the body, delivery of the administered physiologically active substance to the body and absorption of the administered physiologically active substance into the body.

A method was reported in which an anti-epilepsy drug phenytoin was mixed with EUDRAGIT or the like and then subjected to spray lyophilization (spray freeze-drying) to produce porous microcapsules with a size of single micron (Non-Patent Reference 4). According to this method, small microcapsules with a size of single micron can be produced by a spray lyophilization technique. However, since the solid content concentration of the spray solution is as low as 1-3%, there is a problem of low productivity of the lyophilization process. Since both of the two types of capsule materials that are used in this report, i.e., EUDRAGIT and hydroxymethyl propyl cellulose, are limited to medicinal use, there is a problem that this method cannot be used for application to food and feed. Furthermore, the drug used is a low-molecular-weight compound with a molecular weight of 252.27, called phenytoin, which, unlike a peptide or a protein, cannot be applied to a polymer compound with a molecular weight of several thousands to more than several tens of thousands.

A method for forming a complex with a polyelectrolyte is disclosed (Patent Reference 10). Although a protein can be encapsulated according to this method,, there is a problem that degradation of the encapsulated protein cannot be prevented under the pH conditions equivalent to those of animal or human gastric fluid (pH 1.0-1.5). In addition, this production process requires, for example, the steps of mixing an aqueous solution containing lactoferrin and a polymeric acid solution, collecting the resulting complex and further drying the complex, which causes problems of complicated production process and high production cost.

A method for producing sustained-release lactoferrin microparticles by treating acidic polymer gel with an aqueous basic polymer solution was disclosed (Non-Patent Reference 5). According to this method, lactoferrin is encapsulated into a gel such as calcium alginate by a liquid drying technique that uses liquid paraffin containing sorbitan sesquioleate, and then the resultant is treated with a basic polymer. However, there are problems that the liquid paraffin used cannot be used for food production and that the produced microparticles are limited to use for pharmaceutical products. Since microparticles are produced through the complicated steps of: removing moisture by drying under reduced pressure in the liquid drying technique; and washing and again treating the collected microparticles with a basic polymer, there was also a problem of high production cost. Moreover, although the produced microparticles result sustained-release, they are not enteric. Therefore, the content, i.e., lactoferrin, was more likely to be release in a simulated acidic gastric fluid than in a simulated neutral enteric fluid, and thus there is a problem that degradation of lactoferrin contained in the particles cannot be prevented in the stomach.

### PATENT REFERENCES

[Patent Reference 1] Japanese Unexamined Patent Application Publication No. 2002-161050
[Patent Reference 2] WO2006/082824
[Patent Reference 3] Japanese Unexamined Patent Application Publication No. Heisei 11-130697
[Patent Reference 4] Japanese Unexamined Patent Application Publication No. Heisei 9-52847
[Patent Reference 5] Japanese Unexamined Patent Application Publication No. Heisei 5-85941
[Patent Reference 6] Japanese Patent No.3264948
[Patent Reference 7] Japanese Examined Patent Publication No. 2009-520814
[Patent Reference 8] Japanese Examined Patent Publication No. 2007-520202
[Patent Reference 9] Japanese Examined Patent Publication No. 2009-537322
[Patent Reference 10] WO2006/016595

### NON-PATENT REFERENCES

[Non-Patent Reference 1] Bhopatkar et al, Journal of Micro encapsulation, 2005; 22:91-100
[Non-Patent Reference 2] Kanwar JR et al, Nanomedicine (Lond) 2012 7:1521-50
[Non-Patent Reference 3] Lee et al, Bio-Medical Materials and Engineering 21 (2011) 25-36
[Non-Patent Reference 4] Niwa et al, 2009 International Journal of Pharmaceutics, 382: 88-97
[Non-Patent Reference 5] Expert Opin Drug Deliv. 2011 Nov;B(11):1469-79 Drug Dev Ind Pharm. 2010 Aug; 36(8):879-84

### DISCLOSURE OF THE INVENTION

As described above, there has been a demand for a development of a method for producing microparticles having a sufficiently small average particle size in a simple and inexpensive manner.

The present inventors have gone through intensive studies and found that microparticles with an average particle size of 100 µm or less can easily be produced by simultaneously spraying an active element and two or more types of matrix-forming components into contact with each other in the air. In addition, the present inventors also found that the microparticles produced in such a manner shows excellent enteric property, thereby accomplishing the present invention.

Thus, the present invention is as follows.
[1] A method for producing microparticles with a diameter of 100 µm or less, wherein the method comprises the steps of:
   simultaneously spraying (an) active element(s), a matrix-forming component A and a matrix-forming component B that can bind with the matrix-forming component A; and
   collecting microparticles having the active element(s) carried in a polymer structure formed with the bound components A and B.
[2] The method according to [1] above, wherein the active element(s) and the matrix-forming component A are contained in a first solution, and the matrix-forming component B is contained in a second solution.
[3] The method according to [1] above, wherein the active element(s) and the matrix-forming component B are contained in a first solution, and the matrix-forming component A is contained in a second solution.
[4] The method according to [1] above, wherein the active element(s), the matrix-forming component A and the matrix-forming component B are contained in first to third solutions, respectively.
[5] The method according to [1] above, wherein the active element(s) and the matrix-forming component A are contained in a first solution, and the active element(s) and the matrix-forming component B are contained in a second solution.
[6] The method according to any one of [2]-[5] above, wherein the each solution is sprayed from each nozzles.
[7] The method according to any one of [1]-[6] above, wherein the active element(s) is a protein(s) and/or a peptide(s).
[8] The method according to any one of [1]-[6] above, wherein the active element(s) comprises at least one component selected from the group consisting of:
   bovine lactoferrin, human lactoferrin, recombinant bovine lactoferrin, recombinant human lactoferrin, lactoperoxidase, lysozyme, ribonuclease, TGFβ, angiogenin, interferons, interleukins, granular colony-stimulating factor, erythropoietin, lactoferricin, insulin, insulin analogs, insulin derivatives, GLP-1, GLP-1 analogs, GLP-1 derivatives, glucagon luteinizing hormone-releasing hormone, leuprorelin, calcitonin, vasopressin and active fragments thereof.
[9] The method according to any one of [1]-[8] above, wherein the matrix-forming component A comprises a compound(s) having a cationic dissociable group(s).
[10] The method according to any one of [2]-[9] above, wherein the matrix-forming component B comprises a compound(s) having an anionic dissociable group(s).
[11] The method according to any one of [1]-[10] above, wherein the matrix-forming component A comprises at least one component selected from the group consisting of:
   chitosan, chitosan oligosaccharide, polylysine, polyarginine, spermidine, putrescine, lysine, arginine, calcium chloride and calcium lactate.
[12] The method according to [11] above, wherein a component(s) of the matrix-forming component A is in a form of sodium salt, magnesium salt or calcium salt.
[13] The method according to any one of [1]-[12] above, wherein the matrix-forming component B comprises at least one component selected from the group consisting of:
   inositol-6-phosphate, citric acid, alginic acid, low-molecular-weight alginic acid, hyaluronic acid, pectin, carboxymethyl cellulose, carrageenan, aspartic acid, glutamic acid, deoxyribonucleic acid, oligodeoxynucleotide, deoxynucleotide, pyrophosphoric acid, tripolyphosphoric acid, metaphosphoric acid, polyaspartic acid, polylactic acid, polyglutamic acid, malic acid, tartaric acid and succinic acid.
[14] The method according to [13] above, wherein a component(s) of the matrix-forming component B is in a form of sodium salt, magnesium salt or calcium salt.
[15] Microparticles having an average particle size of 100 µm or less, produced by the method according to any one of [1]-[14] above.
[16] Medicine, feedstuff or food comprising the microparticles according to [15] above.
[17] The method according to [1] above, further comprising a step of drying the formed microparticles.
[18] The method according to [17] above, wherein the drying step is carried out at -197°C to +250°C and 0 to atmospheric pressure.

According to the above-described method, microparticles having an average particle size of 100 µm or less can be produced in a simple and inexpensive manner. In addition, the microparticles having an average particle size of 100 µm or less produced according to the above-described method can be used to produce novel medicine, feedstuff or food.

### BRIEF DESCRIPTION OF THE DRAWINGS

Figure 1 is a graph showing the results from dissolution tests of macroparticles produced according to a method employing a conventional technique (solid line) and microparticles produced according to a method of the present invention(dashed line).
Figure 2 is a graph showing a particle size distribution of the microparticles of the present invention.
Figure 3 is a graph showing a particle size distribution of the macroparticles produced according to the method employing the conventional technique.
Figure 4 is the electrophoresis pattern showing the results from SDS-PAGE analysis of the contents recovered from the small intestine of rat models.
Figure 5 is a graph showing a particle size distribution of the microparticles of the present invention.

### BEST MODES FOR CARRYING OUT THE INVENTION

Hereinafter, the present invention will be described in detail. The following embodiments are just examples for illustrating the present invention, and the present invention is not intended to be limited to these embodiments. The present invention may be carried out in various embodiments without departing from the scope of the invention.

All References, unexamined patent applications, patent publications and other patent References cited herein, are incorporated herein by reference. In addition, the present specification incorporates the contents of the specification and drawings of Japanese Patent Application No. 2013-171688 filed on August 21, 2013, which serves as the basis for claiming priority of the present application.

### 1. Method for producing microparticles

The present inventors found that very fine microparticles with an average particle size of 100 µm or less can be formed by simultaneously spraying an active element(s), a matrix-forming component A and a matrix-forming component B that can bind with the matrix-forming component A.

Thus, the present invention provides a method for producing microparticles with a diameter of 100 µm or less (hereinafter, referred to as a "method of the present invention"), wherein the method comprises the steps of:
simultaneously spraying an active element(s), a matrix-forming component A and a matrix-forming component B that can bind with the matrix-forming component A; and
collecting microparticles having the active element(s) carried in a polymer structure formed with the bound components A and B.

According to a method of the present invention, an active element(s), a matrix-forming component A and a matrix-forming component B are preferably contained in solutions or suspensions (hereinafter, "a solution or a suspension" is collectively referred to as a "solution", which means that the term "solution" as used in the present application may include both solution and suspension). However, since the matrix-forming component A and the matrix-forming component B have the property of binding to each other, they need to be contained in separate solutions.

Examples of solutions containing an active element(s), a matrix-forming component A or a matrix-forming component B include the following cases.
(1) A case where the active element(s) and the matrix-forming component A are contained in a first solution, and the matrix-forming component B is contained in a second solution.
(2) A case where the active element(s) and the matrix-forming component B are contained in a first solution, and the matrix-forming component A is contained in a second solution.
(3) A case where the active element(s), the matrix-forming component A and the matrix-forming component B are contained in first to third solutions, respectively.
(4) A case where the active element(s) and the matrix-forming component A are contained in a first solution, and the active element(s) and the matrix-forming component B are contained in a second solution.

In the above-mentioned cases (1)-(4), the active element(s) may be of a single type or multiple types. In particular, in case (4), the active element(s) contained in the first solution and the active element(s) contained in the second solution may be the same or different.

A usual atomizer can be used to spray the solutions.

Herein, the phrase "simultaneous spraying" does not necessarily mean that they are physically sprayed at the same time, and a certain amount of time lag may occur as long as a mist of the mixture of the active element(s), the matrix-forming component A and the matrix-forming component B is formed.

Thus, the step of "simultaneously spraying" may be:
(i) an embodiment where an active element(s), a matrix-forming component A and a matrix-forming component B are mixed after being discharged from the nozzles of the atomizer (hereinafter, referred to as a "post-spray mixing process"); or
(ii) an embodiment where an active element(s), a matrix-forming component A and a matrix-forming component B are mixed before spraying, and the resulting mixture is immediately sprayed (hereinafter, referred to as a "post-mixing spraying process").

### (i) Post-spray mixing process

Examples of a post-spray mixing process will be described based on the cases (1)-(4) above, but the present invention is not limited thereto. Conditions for "simultaneously spraying" are fulfilled the requirements when a second solution (and a third solution) is sprayed while a mist of a first solution stays in the air so that mists of both solutions (or all of the solutions) make contact in the air.

Here, the term "contact" does not necessarily mean that the mists are entirely brought into contact. Accordingly, the state of "contact" as used with the present invention is accomplished as long as the mists are partially brought into contact. This is because microparticles of the present invention can be formed even by partial contact.

Moreover, the phrase "in the air" means that droplets in the mist are in a rising or falling state due to the propellant force and in a free-falling state due to gravity. Specifically, the phrase means that the droplets in the mist are in a state where they are not supported by a reservoir, floor or the like.

Now, in the post-spray mixing process, the respective components are mixed while being contained in the droplets of mists. This mixing is immediately accomplished by bringing the mists of the respective solutions into contact.

The time that takes from spraying to contact is within a second, preferably within 900 milliseconds, within 800 milliseconds, within 700 milliseconds, within 600 milliseconds or within 500 milliseconds.

The active element(s), the matrix-forming component A and the matrix-forming component B are mixed together upon contact, where the matrix-forming components A and B immediately react with each other to form a cross-link. The active element(s) is retained in this cross-linked structure, thereby forming microparticles of the present invention. Specifically, microparticles of the present invention are immediately formed once the active element(s) and the matrix-forming components A and B are brought into contact.

### (ii) Post-mixing spraying process

In the post-mixing spraying process, an active element(s), a matrix-forming component A and a matrix-forming component B are mixed before spraying. Therefore, the matrix-forming components A and B may possibly be initiating the binding reaction at the time of spraying.

However, if the time between mixing and spraying is sufficiently short, the mixture can be sprayed before the binding reaction between components A and B is completed, in which case the binding reaction would be completed after the spraying.

Accordingly, microparticles of the present invention can be formed by the post-mixing spraying process if the time between mixing and spraying is sufficiently short.

Hence, conditions for "simultaneously spraying" are fulfilled the requirements for the post-mixing spraying process as long as microparticles having the active element(s) carried in polymer structures formed with the bound components A and B are formed.

The time that takes between mixing to spraying is less than 100 ms, less than 90 ms, less than 80 ms, less than 70 ms, less than 60 ms, less than 50 ms, less than 40 ms, less than 30 ms, less than 20 ms, less than 10 ms or less than 5ms.

Once the matrix-forming components A and B are mixed, they immedeatly react with each other to form a cross-link. The active element(s) is retained in this cross-linked structure, thereby forming microparticles of the present invention. Specifically, microparticles of the present invention are immedeatly formed once the active element(s) and the matrix-forming components A and B are brought into contact.

Furthermore, the (microscopic) structure of the microparticles of the present invention will specifically be described. One of the characteristics of the microparticles of the present invention is their size. An average particle size of the particles as observed by laser diffractometry or with an electronic microscope is 100 µm or less. The term "average particle size" as used herein refers to a particle size at an integrated value of 50% of the equivalent sphere diameters obtained by a microscopic or laser diffractometry method. Although particles may be produced into various shapes such as a sphere, an ellipsoid, a biconvex lens shape, a hemispherical shape, a partially opened sphere or hemisphere, or a porous body thereof, 90% or more of the microparticles produced by the same production method in the same lot have the same shape.

Additionally, the microparticles of the present invention are characteristic in that an active element(s) is carried in a matrix structure that is formed through reaction between the matrix-forming components A and B.

Preferably, a method of the present invention is carried out by a post-spray mixing process.

Moreover, in any of the above-mentioned cases (1)-(4), the solutions are preferably each sprayed from separate nozzles.

Preferably, the respective solutions are simultaneously sprayed by an atomizer(s) with closely arranged multiple nozzles. Thus, the respective solutions will make contact with each other in mist states.

An example of an atomizer with closely arranged multiple nozzles includes a spray dryer provided with three-fluid nozzles (B290 apparatus from Buchi equipped with nozzles under model number 46555). This spray dryer has nozzles for two types of liquids as well as compressed gas.

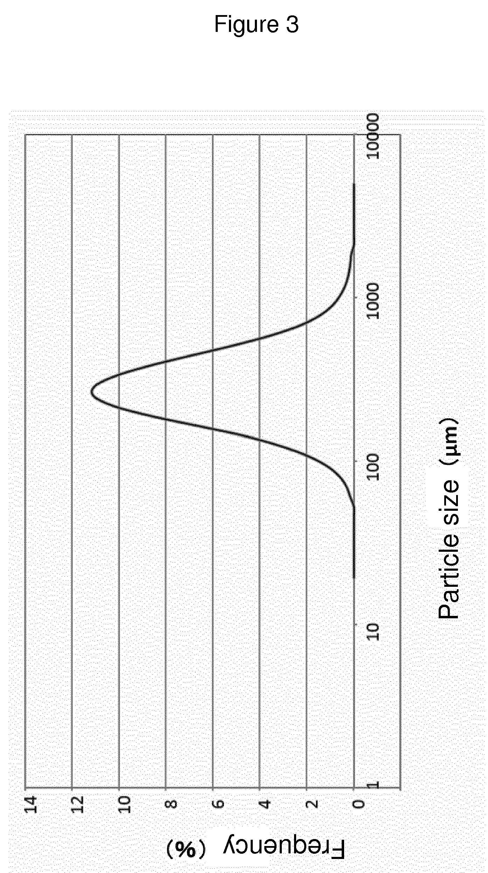

In addition, four-fluid nozzles (for example, Figures 3, 4, and 5) described in Japanese Patent No. 2797080 (page 6, lines 5-47) can also be used. The four-fluid nozzles can spray two types of liquids from two nozzles and gas from the remaining two nozzles.

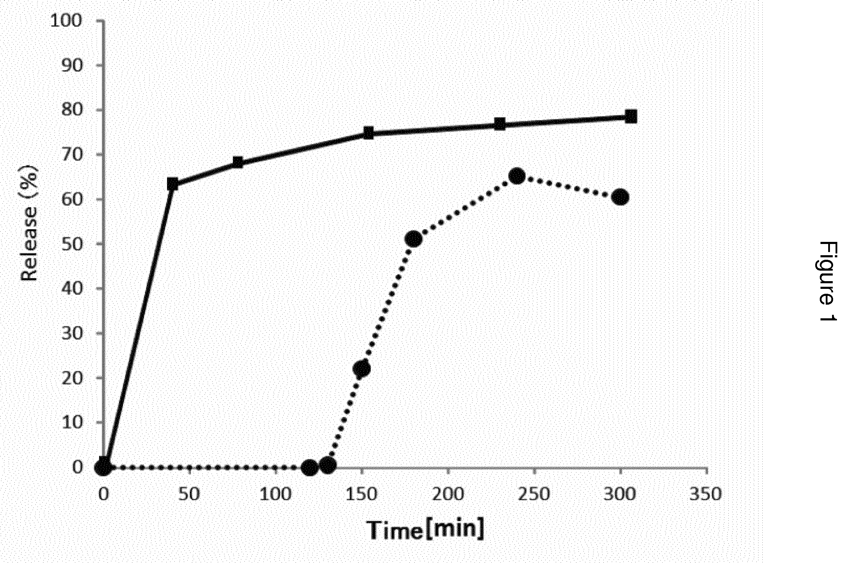

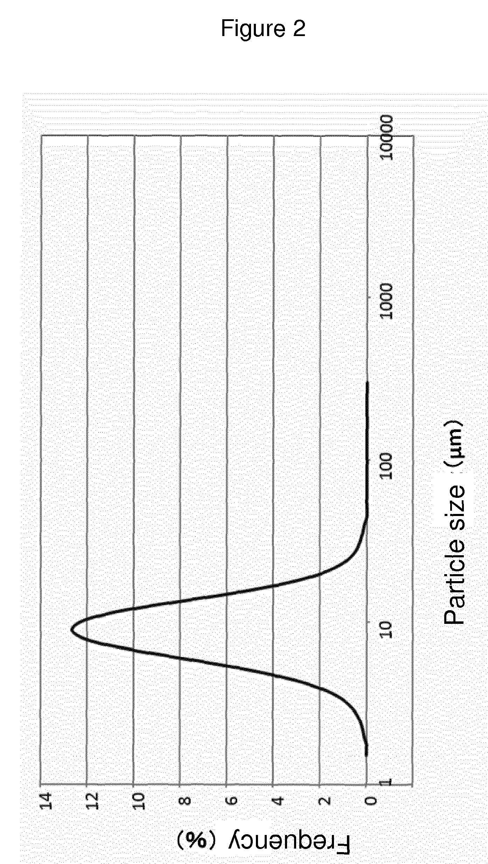

Furthermore, Japanese Unexamined Patent Application Publication No. 2003-117442 (page 5, line 19 to page 8, line 47) describes a method for bringing jets of two types of solutions into contact by allowing them to collide with each other at a collision angle of 45-150° (Figures 1 and 2). The "contact" state of the present invention can also be achieved by this method.

Examples of atomizers that can achieve "simultaneous spraying" as referred to by the present invention include B-290 equipped with nozzles under model number 46555 from Nihon Buchi, as well as MDL-050B and 050C (Fujisaki Electric) equipped with three-fluid and four-fluid nozzles, NL-5 (Ohkawara Kakohki) equipped with TwinJet nozzles RJ10TLM1, and RL-5 (Ohkawara Kakohki) equipped with TwinJet nozzles RJ-10.

In particular, NL-5 (Ohkawara Kakohki) and RL-5 (Ohkawara Kakohki) can be used to easily carry out the post-mixing spraying process.

The formed microparticles may be directly collected or collected after a drying treatment.

The drying process may be simultaneously or concurrently performed with spraying. Specifically, in order to perform the drying process simultaneously or concurrently with spraying, the spraying step is carried out under the same atamspheric conditions for drying the microparticles. For example, the above-mentioned atomizer can be used to simultaneously perform the drying treatment with spraying.

Conditions for the drying treatment may be appropriately selected from -197°C to +250°C. In one embodiment, an inlet temperature of a spray dryer is adjusted to 100-300°C, preferably 120-250°C, and more preferably 150-220°C. An outlet temperature is adjusted to 30°C or higher, preferably 40°C or higher, and more preferably 45°C or higher. In this case, the atmospheric pressure is adjusted to 0-1 atm. While spray drying is usually carried out under the atmospheric pressure conditions (1 atm), spray drying under reduced pressure will be performed under the conditions in which the pressure is reduced with a vacuum pump.

Alternatively, when an active element(s) is a thermally-labile substance, the microparticles may be collected in a precipitated state by cooling immediately after spraying by a method such as spray chilling or spray lyophilization. Alternatively, the cooling may be simultaneously or concurrently performed with spraying. Specifically, in order to perform the cooling process simultaneously or concurrently with spraying, the spraying step is carried out under the same atamspheric conditions for cooling the microparticles.

Conditions for the cooling step would be a temperature of 30-70°C in the case of spray chilling, while, in the case of spray lyophilization, the microparticles are cooled or frozen at a temperature of -196 to 0°C with liquid nitrogen or a cooling equipment or through self-freezing phenomenon by evaporation of water. The atmospheric pressure is adjusted to 0-1 atm. While an atmospheric pressure (1 atm) is employed in the case of spray chilling, a vacuum to an atmospheric pressure is employed in the case of spray lyophilization.

The microparticles of the present invention can be used for various purposes according to a physiological action of an active element.

While an active element used with a method of the present invention is not particularly limited as long as it is a physiologically active compound, it is preferably a protein or a peptide.

Examples of peptide used with a method of the present invention include bovine lactoferrin, human lactoferrin, recombinant bovine lactoferrin, recombinant human lactoferrin, lactoperoxidase, lysozyme, ribonuclease, TGFβ, angiogenin, interferons, interleukins, granular colony-stimulating factor, erythropoietin, lactoferricin, insulin, insulin analogs, insulin derivatives, GLP-1, GLP-1 analogs, GLP-1 derivatives, glucagon luteinizing hormone-releasing hormone, leuprorelin, calcitonin, vasopressin or active fragments thereof.

Moreover, an active element(s) may be a composition containing multiple types of active substances.

While a matrix-forming component A used with a method of the present invention is not particularly limited, it preferably contains one or more compounds having a cationic dissociable group(s).

Examples of such compounds include calcium lactate, calcium chloride, chitosan, low-molecular-weight chitosan, chitosan oligosaccharide, polylysine, polyarginine, spermine, spermidine, putrescine, lysine and arginine.

Moreover, each of these compounds may also be in a form of sodium salt, magnesium salt or calcium salt.

While a matrix-forming component B is not particularly limited as long as it is capable of binding to the matrix-forming component A, it preferably contains a compound having an anionic dissociable group(s).

Examples of such compounds include inositol-6-phosphate, citric acid, alginic acid, low-molecular-weight alginic acid, hyaluronic acid, pectin, carboxymethyl cellulose, carrageenan, aspartic acid, glutamic acid, deoxyribonucleic acid, oligodeoxynucleotide, deoxynucleotide, pyrophosphoric acid, tripolyphosphoric acid, metaphosphoric acid and polyaspartic acid, polylactic acid, polyglutamic acid, malic acid, tartaric acid and succinic acid.

Moreover, each of these compounds may also be in a form of sodium salt, magnesium salt or calcium salt.

A combination and mixed amounts of the matrix-forming components A and B can appropriately be determined by those skilled in the art according to the required particle size of the microparticles and the type of the active element.

Hereinafter, combinations of matrix-forming components A and B will be exemplified, although the present invention is not limited thereto.

When chitosan is contained as a component of a matrix-forming component A, a matrix-forming component B would contain a compound(s) having an anionic dissociable group(s) in the molecule, and the matrix-forming component B preferably contains, as a component, at least one selected from the group consisting of inositol-6-phosphate, citric acid, alginic acid, low-molecular-weight alginic acid, hyaluronic acid, pectin, carboxymethyl cellulose, carrageenan, aspartic acid, glutamic acid, deoxyribonucleic acid, oligodeoxynucleotide, deoxynucleotide, pyrophosphoric acid, tripolyphosphoric acid, metaphosphoric acid, polyaspartic acid, polylactic acid, polyglutamic acid, malic acid, tartaric acid and succinic acid, as well as sodium salt, magnesium salt and calcium salt of these compounds.

When polylysine is contained as a component of a matrix-forming component A, a matrix-forming component B would contain a compound(s) having an anionic dissociable group(s) in the molecule, and the matrix-forming component B preferably contains, as a component, at least one selected from the group consisting of inositol-6-phosphate, citric acid, alginic acid, low-molecular-weight alginic acid, hyaluronic acid, pectin, carboxymethyl cellulose, carrageenan, aspartic acid, glutamic acid, deoxyribonucleic acid, oligodeoxynucleotide, deoxynucleotide, pyrophosphoric acid, tripolyphosphoric acid, metaphosphoric acid, polyaspartic acid, polylactic acid, polyglutamic acid, malic acid, tartaric acid and succinic acid, as well as sodium salt, magnesium salt and calcium salt of these compounds.

When alginic acid is contained as a component of a matrix-forming component B, a matrix-forming component A would contain a compound(s) having a cationic dissociable group(s) in the molecule, and the matrix-forming component A preferably contains, as a component, at least one selected from the group consisting of calcium lactate, calcium chloride, chitosan, low-molecular-weight chitosan, chitosan oligosaccharide, polylysine, polyarginine, spermine, spermidine, putrescine, lysine and arginine.

When pectin is contained as a component of a matrix-forming component B, a matrix-forming component A would contain a compound(s) having a cationic dissociable group(s) in the molecule, and the matrix-forming component A preferably contains, as a component, at least one selected from the group consisting of chitosan, low-molecular-weight chitosan, chitosan oligosaccharide, polylysine, polyarginine, spermine, spermidine, putrescine, lysine and arginine.

The matrix-forming components A and B may each have one type of compound as the component, or may have a mixture of several compounds as the components. For example, a matrix-forming component A may contain chitosan and calcium lactate while a matrix-forming component B may contain sodium alginate and inositol-6-phosphate so that the matrix-forming components A and B will have a combination of each two components.

Alternatively, matrix-forming components A and B may have a combination of one plus multiple components. An exemplary case of such a combination may include a case where a matrix-forming component A contains chitosan as a component while a matrix-forming component B contains sodium alginate, inositol-6-phosphate and oligodeoxynucleotide.

Moreover, each of the matrix-forming components may contain similar compounds with different molecular weights. Exemplary cases include a case where a matrix-forming component A contains calcium lactate while a matrix-forming component B contains alginic acid and a low-molecular-weight alginic acid, and a case where a matrix-forming component A contains chitosan and a low-molecular-weight chitosan while a matrix-forming component B contains inositol-6-phosphate.

The mixed ratio of the matrix-forming components A and B (the ratio of the whole mixture if the matrix-forming component is a mixture) may be, for example, within a range of 1:1000 to 100:1 at a molar ratio, preferably 1:100 to 10:1 at the total molar ratio of the dissociable group(s) of each of the matrix-forming components A and B, and most preferably:10 to 5:1 at the total molar ratio of the dissociable group(s).

A solvent that is used when an active element, a matrix-forming component A and a matrix-forming component B are contained in solutions is not particularly limited as long as it does not denature or deactivate the active element, and may be either an aqueous solvent or an organic solvent. Examples of preferable solvents include water, acetic acid, ethanol and a mixed solution containing them in arbitrary proportions.

When an active element is contained in a solution, a concentration thereof is 0.01-50% (w/v), 0.01-50% (w/v), 0.1-50% (w/v), 0.5-50% (w/v), 1.0-50% (w/v), 2.0-50% (w/v) 3.0-50% (w/v), 4.0-50% (w/v), 5.0-50% (w/v), 6.0-50% (w/v), 7.0-50% (w/v), 8.0-50% (w/v), 9.0-50% (w/v) or 10-50% (w/v).

When a matrix-forming component A is contained in a solution, a concentration thereof is 0.01-50% (w/v), 0.1-45% (w/v) or 0.5-40% (w/v).

When a matrix-forming component B is contained in a solution, a concentration thereof is 0.01-50% (w/v), 0.1-45% (w/v) or 0.5-40% (w/v).

When producing microparticles of the present invention, in addition to an active element(s), a matrix-forming component A and a matrix-forming component B, an additive such as a stabilizer may also be added. Examples of additives include amino acids (for example, arginine, lysine, phenylalanine, etc.), glucose, sorbitol, glycerol, mannitol, sodium phosphate, propylene glycol, dextran (for example 18-82kD), polyvinylpyrrolidone (PVP), heparin, gelatin (types A and B), hydroxyethylated starch (HES), dextran sulfate, polyphosphoric acid,, polyglutamic acid, polyaspartic acid, polylactic acid, konjac, glucomannan, pullulan, gelatin, shellac, zein, pectin, carboxymethyl cellulose, methylcellulose, hydroxypropyl cellulose, hydroxypropyl methylcellulose, water-soluble soybean polysaccharides, guar gum, xanthan gum, tamarind gum, carrageenan, Eudragit (registered trademark), Carbopol (registered trademark) and hydroxyethyl methacrylate. These additives may be added by being sprayed simultaneously with the active element(s) and the matrix-forming components A and B, or added to a solution containing the active element or the matrix-forming component A or B prior to spraying.

### 2. Microparticles and uses thereof

The microparticles of the present invention are characterized in that they contain at least an active element, a matrix-forming component A and a matrix-forming component B, and in that their average particle size is 100 µm or less, 90 µm or less, 80 µm or less, 70 µm or less, 60 µm or less, 50 µm or less, 40 µm or less, 30 µm or less, 20 µm or less or 10 µm or less.

The active element(s) and the matrix-forming components A and B have already been described above. The microparticles may also contain additives in addition to the active element(s) and the matrix-forming components A and B. Additives have also been described above.

The microparticles of the present invention can be used for various purposes according to the type of the active element.

For example, the microparticles of the present invention may be used as medicine, or as a food additive or a feedstuff additive.

In the case where the microparticles of the present invention are used as medicine, other components (such as a carrier or an excipient) may be added to microparticles so as to give a form of a pharmaceutical composition (hereinafter, referred to as a "pharmaceutical composition of the present invention").

A form of administration of a pharmaceutical composition of the present invention is not particularly limited as long as the form of administration is pharmaceutically acceptable, which may be selected according to the treatment procedure. Preferably, it is oral administration, sublingual administration, nasal administration, pulmonary administration, gastrointestinal administration, transdermal administration, ophthalmic administration, intravenous injection, subcutaneous injection, intramuscular injection, intraperitoneal injection, local injection, surgical implantation or the like, where oral administration is particularly preferable.

A pharmaceutical composition of the present invention may be a solid form such as granules, a capsule, a tablet or powder, a liquid agent such as a solution, a suspension or an emulsion, or a semiliquid preparation such as ointment, cream or paste. Alternatively, it may be an inhaler, an adhesive patch, a spray agent, a topical agent or medicated toothpaste.

Besides the microparticles of the present invention and the above-described carrier, the pharmaceutical composition of the present invention may optionally be added with other pharmaceutically acceptable components. Examples of other pharmacologically acceptable components include, but not limited to, excipients, binders, disintegrants, antioxidants, preservatives, adjuvants, lubricants, sweeteners and aroma chemicals. Examples of other pharmaceutically acceptable components include emulsified adjuvants (for example, fatty acids with carbon numbers of 6-22 and pharmaceutically acceptable salts thereof, albumin, dextran), stabilizing agents (for example, cholesterol, phosphatidic acid), tonicity agents (for example, sodium chloride, glucose, maltose, lactose, sucrose, trehalose) and pH adjusters (for example, hydrochloric acid, sulfuric acid, phosphoric acid, acetic acid, sodium hydroxide, potassium hydroxide, triethanolamine). One or more types of them can be used. The content of such an additive(s) in a composition of the present invention is suitably 90% by weight or less, preferably 70% by weight or less, and more preferably 50% by weight or less.

A pharmaceutical composition of the present invention can be prepared by adding the microparticles of the present invention to a dispersion of the carrier and then appropriately stirring. The additives may be added in a suitable step before or after the addition of the microparticles of the present invention. An aqueous solvent that can be used upon preparing a pharmaceutical composition of the present invention is not particularly limited as long as it is pharmaceutically acceptable, and examples include electrolyte fluids such as injectable water, injectable distilled water and physiological saline, and carbohydrate solutions such as dextrose solution and maltose solution. Conditions such as pH and temperature in this case may appropriately be selected by those skilled in the art.

The composition of the present invention may be, for example, a liquid agent or a lyophilized preparation thereof. The lyophilized preparation can be prepared by liophylizing the microparticles that are in a form of a liquid agent according to a common method. For example, a composition of the present invention in a form of a liquid agent can be lyophilized with the steps of performing suitable sterilization and dispensing a predetermined amount of it in a vial bottle, which is subjected to pre-freezing under the condition of about -40 to -20°C for around 2 hours, subjected to primary drying at about 0-10°C under reduced pressure, and subsequently subjected to secondary drying at about 15-25°C under reduced pressure. In general, air inside the vial is replaced with nitrogen gas and the vial is capped, thereby obtaining a lyophilized preparation of the composition of the present invention.

The lyophilized preparation of the composition of the present invention can be used by resolving it by adding any suitable solution (resolving solution). Examples of such resolving solutions include injectable water, physiological saline and other general infusion solutions. Although the volume of this resolving solution differs depending on the use and is not particularly limited, a voloume that is 0.5-2 times the volumeof the solution before lyophilization or 500 ml or less is suitable.

With respect to a dosage of the pharmaceutical composition of the present invention, it is preferably prepared considering the type of the microparticles of the present invention contained, the dosage form, the conditions of the patient such as age and weight, the route of administration, and the nature and severity of the disease. Generally, the daily amount of the active element(s) in the microparticles of the present invention is in a range of 0.1mg-20g/human for an adult, and preferably in a range of 1 mg-1 g. These values may also differ depending on the type of the active element(s), the type of the target disease, the form of administration and the target molecule. Accordingly, a dose less than these values may be sufficient in some cases while a required dose, to the contrary, may be greater than these values in some cases.

For example, when microparticles having bovine lactoferrin or human lactoferrin as an active element are orally administered to an adult with a weight of 60 kg, it may be administered 1-4 times, preferably 1-2 times a day with the dosage at each time being 0.1-500 mg, and preferably 1-300 mg.

In addition, the microparticles of the present invention exhibit an excellent enteric property. In particular, components that are absorbed from the intestine such as lactoferrin are expected to give higher effect if they are delivered to the intestine without being degraded by the gastric acid. Therefore, the microparticles of the present invention are useful for preparing a medicine that contains, as an active element(s), a substance(s) that acts in the intestine or a substance(s) that acts after being absorbed from the intestine.

Other than medicine, the microparticles of the present invention can also be used as a raw material or an additive for supplements, cosmetics, food and feedstuff. When they are used as a raw material or an additive, the particles and other materials may directly be mixed together or may be mixed after a heat sterilization treatment.

For example, when microparticles having bovine lactoferrin as an active element are used as a raw material for medicine, supplements or food, they can bring about an effect such as an antibacterial effect, an antiviral effect, immune enhancement, protection against infection, an anticancer effect, blood-pressure regulation, an opioid-like effect, improvement in insomnia, an anti-anxiety effect, improvement in cognitive symptoms, improvement in memory impairment, improvement in lipid metabolism, antioxidation, anti-aging, an anti-inflammatory effect, stimulation of iron absorption, improvement in dry eye and pain relief. When used as feedstuff, the microparticles can bring about, in addition to the above-mentioned effects as a raw material for medicine, supplements and food, effects such as growth promotion, development promotion, improvement in yield, improvement in meat quality, improvement in taste, improvement in flavor and improvement in color tone.

Examples of forms of supplements include granules, tablets, capsules, an adhesive patch, powder and liquid agents.

Examples of cosmetics include a spraying agent, a cream agent, a powder agent, a mask agent, an adhesive patch, emulsion, skin lotion, soap, shampoo, body shampoo, toothpaste and cleanser.

Examples of food include pudding, jelly, konjac jelly, cream, cream portion, butter, fat and oil, spice, fluid diet, solid nutritious food, cold beverage, alcoholic beverage, sports-drink, mineral water, drinking water, energy drink, milk beverage, modified milk for infant, modified powdered milk for infant, creaming powder, fermented milk, fruit juice, vegetable juice, carbonated drink, yogurt, mayonnaise, dressing, tomato ketchup, seasoning, vinegar, sauce, soy sauce, sweet *sake,* sweet *sake-*like seasoning, ice cream, ice cream mix, whippy ice cream mix, whipped cream, ices, shaved ice, shaved ice syrup, gelato, frozen yogurt, caramel, candy, gummi candy, biscuit, rice cracker, rice cake, bread, bread mix, cake, doughnut, waffle, bagel, potato chips, chocolate, canned food, bottled food, minced product, processed meat, sausage, fish meat sausage, minced product, ham, jam, peanut butter, soybean curd, fermented soybean paste, konjac, konjac noodle, artificial rice, agar, dry noodle, raw noodle, semi-raw noodle, instant noodle, instant soup, retort pouch food, gelator, thickener for people with swallowing difficulty, food for people with swallowing difficulty, baby food, instant coffee, bagged tea, bagged green tea, additives for rice cooker, frozen meals, sandwich, boxed lunch, rice ball, rice seasoning, pickles, *natto* and margarine.

Examples of feedstuff include feed for pet animals, feed for livestock, feed for racehorses, feed for fish, feed for insects, feed for reptiles, feed for amphibians, feed for experimental animals, feed for zoo animals and feed for birds.

### EXAMPLES

Hereinafter, the present invention will specifically be described by means of examples, although the scope of the present invention is not limited to these examples.

### [Example 1] (Active element is solution; pre-mixing spraying process)

Chitosan (from Koyo Chemical) was dissolved in 1% acetic acid to prepare 667 g of 1.5% chitosan. To this solution, 333 ml of 10% bovine lactoferrin (from Morinaga Milk) was added to prepare a spray stock solution. To 50% phytic acid solution (Tsuno Food Industrial Co., Ltd.), sodium hydroxide was added to adjust pH to 6, thereby preparing 667 g of another spray stock solution with a final phytic acid concentration of 6%. NL-6 manufactured by Ohkawara Kakohki was equipped with RJ10-TLM1 nozzles and these two solutions were introduced into the spray tower through separate liquid feed means. Spraying was carried out at an airflow volume of 87 m³/h, an atomization air volume at the nozzle of 9 m³/h and an inlet temperature of 200°C. About 5 g of dry fine powder containing bovine lactoferrin as a physiologically active component was obtained.

### [Example 2] (Active element is solution; pre-mixing spraying process)

Chitosan (from Koyo Chemical) was dissolved in 1% acetic acid to prepare 667 g of 0.15% chitosan. To this solution, 333 ml of 1% bovine lactoferrin (from Morinaga Milk) was added to prepare a spray stock solution. To 50% phytic acid solution (Tsuno Food Industrial Co., Ltd.), sodium hydroxide was added to adjust pH to 6, thereby preparing 667 g of another spray stock solution with a final phytic acid concentration of 0.6%. Spraying was carried out while other conditions were the same as those in Example 1, thereby obtaining about 1 g of dry fine powder containing bovine lactoferrin as a physiologically active component.

### [Example 3] (Active element is solution; pre-mixing spraying process)

10 g sodium alginate (under model number 31130-95 from Nacalai Tesque) was dissolved in 500 ml of water. To this solution, 500 ml of 4.6% bovine lactoferrin was added to prepare a spray stock solution. As another spray stock solution, 1000 ml of 2.5% calcium lactate solution was prepared. Spraying was carried out while other conditions were the same as those in Example 1, thereby obtaining about 5 g of dry fine powder containing bovine lactoferrin as a physiologically active component.

### [Example 4] (Active element is solution; post-spray mixing process)

Chitosan (from Koyo Chemical) was dissolved in 1% acetic acid to prepare 50 ml of 1.5% chitosan. To this solution, 25 ml of 1% bovine lactoferrin (from Morinaga Milk) was added to prepare a spray stock solution. To 50% phytic acid solution (Tsuno Food Industrial Co., Ltd.), sodium hydroxide was added to adjust pH to 6, thereby preparing 75 g of another spray stock solution with a final phytic acid concentration of 6%. B-290 manufactured by Nihon Buchi was equipped with three-fluid nozzles, and these two solutions were introduced into the spray tower through separate liquid feed means. Spraying was carried out under the conditions where an inlet temperature was 150°C. About 1 g of dry fine powder containing bovine lactoferrin as a physiologically active component was obtained.

### [Example 5] (Active element is solution; post-spray mixing process)

8 g sodium alginate (under model number 31130-95 from Nacalai Tesque) was dissolved in 400 ml of water. To this solution, 400 ml of 1.7% bovine lactoferrin was added to prepare a spray stock solution. As another spray stock solution, 800 ml of 2.5% calcium lactate solution was prepared. MDL-050M from Fujisaki Electric was equipped with four-fluid straight edge nozzles SE4003, and these two solutions were introduced into the spray tower at a speed of 15ml/min. through separate liquid feed means. Spray drying was carried out at an inlet temperature of 200°C, an air-intake volume of 1 m³/min. and nozzle air of 45NL/min. About 10 g of dry fine powder containing bovine lactoferrin as a physiologically active component was obtained.

### [Example 6] (Active element is solution; post-spray mixing process)

8 g sodium alginate (under model number 31130-95 from Nacalai Tesque) was dissolved in 400 ml of water. To this solution, 500 g of phytic acid with a final concentration of 6% and 100 ml of 10% bovine lactoferrin were dissolved to prepare about 1000 ml of a spray drying stock solution. 1000 ml of another spray stock solution of 2.5% calcium lactate solution and 1.5% chitosan was prepared. MDL-050M from Fujisaki Electric was equipped with four-fluid straight edge nozzles SE4003, and these two solutions were introduced into the spray tower at a speed of 15ml/min. through separate liquid feed means. Spraying was carried out while other conditions were the same as those in Example 4, thereby obtaining about 10 g of dry fine powder containing bovine lactoferrin as a physiologically active component.

### [Example 7] (Active element is solution; post-spray mixing process)

To 4 L of 5% chitosan solution, 2 L of 10% bovine lactoferrin was added to prepare about 6 L of a spray drying stock solution. 6 L of 4% phytic acid was prepared. MDL-050M from Fujisaki Electric was equipped with four-fluid straight edge nozzles SE4003, and these two solutions were introduced into the spray tower at a speed of 15ml/min. through separate liquid feed means. Spraying was carried out while other conditions were the same as those in Example 4, thereby obtaining about 500 g of dry fine powder containing bovine lactoferrin as a physiologically active component.

### [Example 8]

To 200 g of dry fine powder containing bovine lactoferrin as a physiologically active component produced according to the same method as Example 6, 456g of lactose, 160 g of crystalline cellulose (trade name: Avicel), 16 g of carboxymethyl cellulose/calcium salt and 8 g of sucrose fatty acid ester were added. The resulting mixture was pulverized with a mixer so as to obtain powder that passes through 100 mesh. This mixed powder was subjected to tableting using a tableting machine to produce tablets.

### [Example 9]

To 200 g of dry fine powder containing bovine lactoferrin as a physiologically active component produced according to the same method as Example 6, 50 g of water and 50 g of ethanol were added. The resultant was kneaded in a mortar, then transferred into a stainless-steel kitchen strainer and pushed in with a pestle. The resulting particles were air dried to produce about 150 g of granules.

### [Example 10]

200 g of dry fine powder containing bovine lactoferrin as a physiologically active component produced according to the same method as Example 6, was mixed with 1 kg of condensed milk whey powder to produce 1.2 kg of a whey protein-based muscle-building nutritional supplement.

### [Example 11] (Active element is solution; post-spray mixing process)

To 25 ml of 1% bovine lactoferrin (from Morinaga Milk), porcine pepsin (from Sigma-Aldrich) was added to obtain a final concentration of 0.02%. The resultant was heated at 37°C for 2 hours and then at 68°C for 30 minutes. To this suspension, 50 ml of 1.5% chitosan (from Koyo Chemical) dissolved in 1% acetic acid was added to prepare a spray stock solution. To 50% phytic acid solution (Tsuno Food Industrial Co., Ltd.), sodium hydroxide was added to adjust pH to 6, thereby preparing 75 g of another spray stock solution with a final phytic acid concentration of 6%. B-290 manufactured by Nihon Buchi was equipped with three-fluid nozzles and these two solutions were introduced into the spray tower through separate liquid feed means. Spraying was carried out under the conditions where an inlet temperature was 150°C. About 1 g of dry fine powder containing a bovine lactoferrin-degrading peptide as a physiologically active component was obtained.

### [Example 12] (Active element is solution; post-spray mixing process)

To 25 ml of 1% recombinant human lactoferrin (from Ventria, US), 50 ml of 1.5% chitosan (from Koyo Chemical) dissolved in 1% acetic acid was added to prepare a spray stock solution. To 50% phytic acid solution (Tsuno Food Industrial Co., Ltd.), sodium hydroxide was added to adjust pH to 6, thereby preparing 75 g of another spray stock solution with a final phytic acid concentration of 6%. B-290 manufactured by Nihon Buchi was equipped with three-fluid nozzles and these two solutions were introduced into the spray tower through separate liquid feed means. Spraying was carried out under the conditions where an inlet temperature was 150°C. About 1 g of dry fine powder containing recombinant human lactoferrin as a physiologically active component was obtained.

### [Example 13] (Active element is solution; post-spray mixing process)

To 25 ml of 1% human insulin (from Sigma-Aldrich), 50 ml of 1.5% chitosan (from Koyo Chemical) dissolved in 1% acetic acid was added to prepare a spray stock solution. To 50% phytic acid solution (Tsuno Food Industrial Co., Ltd.), sodium hydroxide was added to adjust pH to 6, thereby preparing 75 g of another spray stock solution with a final phytic acid concentration of 6%. B-290 manufactured by Nihon Buchi was equipped with three-fluid nozzles and these two solutions were introduced into the spray tower through separate liquid feed means. Spraying was carried out under the conditions where an inlet temperature was 150°C. About 1 g of dry fine powder containing human insulin as a physiologically active component was obtained.

### [Test Example 1]

Macroparticles were produced bya conventional method in order to compare their properties with those of the microparticles of the present invention. Macroparticles containing insulin as a physiologically active component were produced following Bio-Medical Materials 21 25-36 2011. Specifically, 3% insulin was dispersed in 3% chitosan dissolved in 1% acetic acid solution to give Stock solution 1. Stock solution 1 was introduced into a syringe equipped with a needle with a diameter of 0.241 mm, and dropped as droplets into a 6% phytic acid solution (pH 6, 25°C) while slowly stirring with a magnetic stirrer. The macroparticles were washed with water and then subjected to lyophilization.

### [Test Example 2]

This method is a test carried out for examining elution characteristics of the microparticles or the macroparticles *in vitro*. Following Bio-Medical Materials 21 25-36 2011, a simulated gastric fluid and a simulated intestinal fluid were prepared. Specifically, the simulated gastric fluid was obtained by dissolving 2 g of sodium chloride and 7 ml of 35% hydrochloric acid and adjusting the resultant to be 1 L with distilled water. The simulated intestinal fluid was obtained by dissolving 250 ml of 0.2M KH₂PO₄ and 118 ml of 0.2N NaOH and adjusting the resultant to be 1 L (pH6.8). The particles to be assessed were incubated with the simulated gastric fluid at 37°C for 2 hours while rotating at 80 rpm, and then with the simulated intestinal fluid at 37°C for another 2 hours or longer. The drug eluted into each of the simulated solutions was allowed to pass through a 0.45 µm filter and applied to reversed-phase HPLC for an analysis.

The microparticles of Example 4 and the macroparticles produced in Test Example 1 were used to examine the elution characteristics. The results are shown in Figure 1. With respect to the conventional macroparticles produced in Test Example 1, more than half of the insulin has eluted in the simulated gastric fluid and scarcely eluted in the subsequent simulated intestinal fluid. Specifically, suppression of the release of the encapsulated insulin was insufficient under an acidic condition corresponding to the gastric fluid. On the other hand, with respect to the microparticles of Example 4 of the present invention, the encapsulated lactoferrin was scarcely eluted in the simulated gastric fluid and mostly eluted in the subsequent simulated intestinal fluid. Accordingly, the conventional macroparticles and the microparticles of the present invention showed completely different eluting properties. The microparticles of the present invention were found to show higher stability in the stomach. The microparticles and food of Examples 1-14 were also analyzed and they gave almost same results.

Figure 1 shows the results from the eluting tests of the microparticles of Example 4 and Test Example 1. In Figure 1, the horizontal axis represents eluting time (min), the vertical axis represents eluting rate (%), the dashed line represents eluting of the microparticles of the present invention, and the solid line represents eluting of the macroparticles of the conventional method.

### [Test Example 3]

The particle size distributions of the microparticles of Example 4 and the macroparticles produced in Test Example 1 were examined by a laser diffraction/scattering particle size distribution measurement. The results are shown in Figures 2 and 3.

In Figure 2, the horizontal axis represents particle size (µm), the vertical axis represents frequency (%), and the black line represents the particle size distribution of the microparticles of the present invention.

In Figure 3, the horizontal axis represents particle size (µm), the vertical axis represents frequency (%), and the black line represents the particle size distribution of the macroparticles produced by the conventional method.

While the average particle size of the microparticles of the present invention was 8 µm with the largest particle size being 100 µm or less, the average particle size of the macroparticles according to the conventional method was several-hundreds of µm with the largest particle size being at least 1,000 µm. An average particle size as used herein refers to a particle size at an integrated value of 50% of the equivalent sphere diameters obtained by the laser diffraction/scattering particle size distribution measurement.

### [Test Example 4]

Improvements in stability of the physiologically active substances in the body after administering the particles into animals, absorption into the body, and delivery into the body were examined.

A lactoferrin control or the microparticles produced in Example 6 dissolved or suspended in 100 mM HCl were administered to 10-week-old fasted rats F344 using a gastric tube. The dosage was 50 mg lactoferrin per kilogram of rat weight. The contents from the small intestines were recoverd after 30 minutes administration. The recovered small intestinal contents were two-fold diluted with an SDS-PAGE sample buffer. The supernatants were subjected to heat denaturation followed by SDS-PAGE.

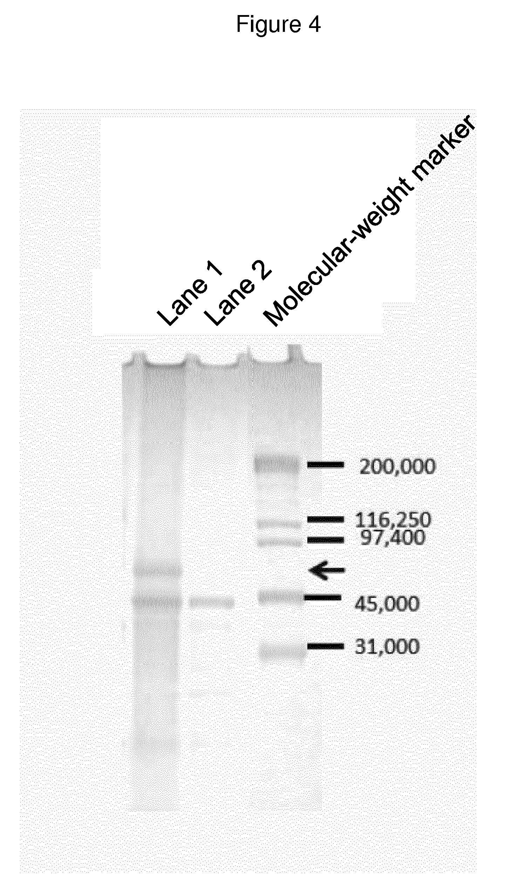

The results from the electrophoresis are shown in Figure 4. Lane 1 shows migration of the small intestinal contents from the rat administerd the microparticulated lactoferrin. Lane 2 shows migration of the small intestinal contents from the rat administered the lactoferrin control. The figures show the size of the molecular weight markers, and the arrow shows the location of the intact lactoferrin.

As shown in Figure 4, intact lactoferrin was observed only in the case where the microparticlated lactoferrin was administered. The microparticles were found to improve stability of the physiologically active substances in the body after administration into animals, absorption into the body, and delivery into the body.

### [Example 14] (Active element is suspension; pre-spray mixing process)

To 50% phytic acid solution (from Tsuno Food Industrial Co., Ltd.), sodium hydroxide was added to adjust pH to 6, and the resultant was diluted with water to prepare 100 ml of 12% phytic acid solution. This solution was mixed with 100 ml of 100% ethanol (from Wako Pure Chemical Industries) to give Stock solution 1. A solution was prepared by dissolving bovine lactoferrin into 1000 ml of 125 mM NaCl to a final concentration of 1%. To this solution, 1000 ml of ethanol was added at once, which was vigorously stirred to prepare a lactoferrin suspension. The suspension was centrifuged at 6000 g. The precipitation was collected and resuspended in the previously added Stock solution 1 to prepare a spray stock solution. Chitosan (from Yaizu Suisankagaku Industry) was dissolved in 1% acetic acid to prepare 200 ml of 1.5% chitosan spray stock solution. NL-6 manufactured by Ohkawara Kakohki was equipped with RJ10-TLM1 nozzles and these two solutions were introduced into the spray tower through separate liquid feed means. The suspension was stirred with a magnetic stirrer so that no sediment was formed in the spray. Spraying was carried out at an airflow volume of 84 m³/h, an atomization air volume at the nozzle of 8 m³/h and an inlet temperature of 200°C. About 8 g of dry fine powder containing bovine lactoferrin as a physiologically active component was obtained.

### [Example 15] (Active element is suspension; post-spray mixing process)

To a 50% phytic acid solution (Tsuno Food Industrial Co., Ltd.), sodium hydroxide was added to adjust pH to 6, and the resultant was diluted with water to prepare 100 ml of 12% phytic acid solution. This solution was mixed with 100 ml of 100% ethanol (from Wako Pure Chemical Industries) to give Stock solution 1. A solution was prepared by dissolving bovine lactoferrin into 1000 ml of 125 mM NaCl to a final concentration of 1%. To this solution, 1000 ml of ethanol was added at once, which was vigorously stirred to prepare a lactoferrin suspension. The suspension was centrifuged at 6000 g. The precipitation was collected and resuspended in the previously added Stock solution 1 to prepare a spray stock solution. Chitosan (from Yaizu Suisankagaku Industry) was dissolved in 1% acetic acid to prepare 200 ml of 1.5% chitosan spray stock solution. MDL-050M manufactured by Fujisaki Electric was equipped with four-fluid straight edge nozzles SE4003 and these two solutions were introduced into the spray tower at a speed of 10 ml/min. through separate liquid feed means. Spray drying was performed at an inlet temperature of 150°C, an air-intake volume of 1m³/min., and nozzle air of 45 NL/min. The suspension was stirred with a magnetic stirrer so that no sediment was formed in the spray. About 12 g of dry fine powder containing bovine lactoferrin as a physiologically active component was obtained.

### [Test Example 5]

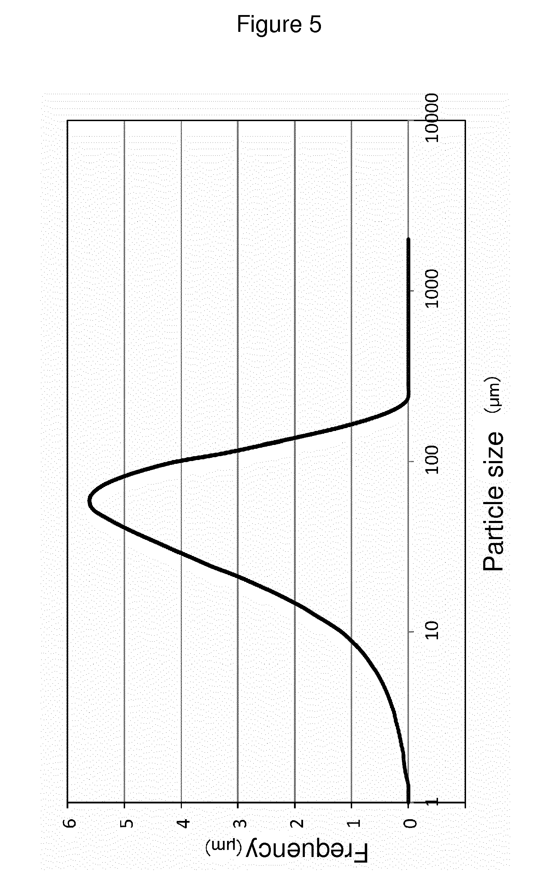

The particle size distribution of the microparticles of Example 15 was examined by a laser diffraction/scattering particle size distribution measurement. The results are shown in Figure 5. In Figure 5, the horizontal axis represents particle size (µm), the vertical axis represents frequency (%), and the black line represents the particle size distribution. The average particle size of the microparticles of Example 15 was 48 µm. Although the particle size of the microparticles of Example 15 was larger than the average particle size (8µm) of the microparticles produced in Example 4 and analyzed in Test Example 3, the average particle size was 48 µm which was still 100 µm or less, where particles of less than 100 µm made up 89% of the entire particles. Specifically, most of the particles obtained in Example 15 had particle sizes of 100 µm or less. The microparticles of Example 14 were also analyzed and gave almost same results. An average particle size as used herein refers to a particle size at an integrated value of 50% of the equivalent sphere diameters obtained by the laser diffraction/scattering particle size distribution measurement.

### INDUSTRIAL APPLICABILITY

According to the method of the present invention, microparticles having an average particle size of 100 µm or less can be produced in a simple and inexpensive manner. In addition, the microparticles having an average particle size of 100 µm or less produced by the descrived methods can be used to produce novel medicine, feedstuff or food.

## Claims

1. A method for producing microparticles with a diameter of 100 µm or less, wherein the method comprises the steps of:
simultaneously spraying an active element, a matrix-forming component A and a matrix-forming component B that can bind with the matrix-forming component A; and
collecting microparticles having the active element carried in a polymer structure formed with the bound components A and B.

2. The method according to Claim 1, wherein the active element and the matrix-forming component A are contained in a first solution, and the matrix-forming component B is contained in a second solution.

3. The method according to Claim 1, wherein the active element and the matrix-forming component B are contained in a first solution, and the matrix-forming component A is contained in a second solution.

4. The method according to Claim 1, wherein the active element, the matrix-forming component A and the matrix-forming component B are contained in first to third solutions, respectively.

5. The method according to Claim 1, wherein the active element and the matrix-forming component A are contained in a first solution, and the active element and the matrix-forming component B are contained in a second solution.

6. The method according to any one of Claims 2-5, wherein the each solution is sprayed from each nozzle.

7. The method according to any one of Claims 1-6, wherein the active element is a protein and/or a peptide.

8. The method according to any one of Claims 1-6, wherein the active element comprises at least one component selected from the group consisting of:
bovine lactoferrin, human lactoferrin, recombinant bovine lactoferrin, recombinant human lactoferrin, lactoperoxidase, lysozyme, ribonuclease, TGFβ, angiogenin, interferons, interleukins, granular colony-stimulating factor, erythropoietin, lactoferricin, insulin, insulin analogs, insulin derivatives, GLP-1, GLP-1 analogs, GLP-1 derivatives, glucagon luteinizing hormone-releasing hormone, leuprorelin, calcitonin, vasopressin and active fragments thereof

9. The method according to any one of Claims 1-8, wherein the matrix-forming component A comprises a compound having a cationic dissociable group.

10. The method according to any one of Claims 2-9, wherein the matrix-forming component B comprises a compound having an anionic dissociable group.

11. The method according to any one of Claims 1-10, wherein the matrix-forming component A comprises at least one component selected from the group consisting of:
chitosan, chitosan oligosaccharide, polylysine, polyarginine, spermidine, putrescine, lysine, arginine, calcium chloride and calcium lactate.

12. The method according to Claim 11, wherein a component of the matrix-forming component A is in a form of sodium salt, magnesium salt or calcium salt.

13. The method according to any one of Claims 1-12, wherein the matrix-forming component B comprises at least one component selected from the group consisting of:
inositol-6-phosphate, citric acid, alginic acid, low-molecular-weight alginic acid, hyaluronic acid, pectin, carboxymethyl cellulose, carrageenan, aspartic acid, glutamic acid, deoxyribonucleic acid, oligodeoxynucleotide, deoxynucleotide, pyrophosphoric acid, tripolyphosphoric acid, metaphosphoric acid, polyaspartic acid, polylactic acid, polyglutamic acid, malic acid, tartaric acid and succinic acid.

14. The method according to Claim 13, wherein a component of the matrix-forming component B is in a form of sodium salt, magnesium salt or calcium salt.

15. Microparticles having a diameter of 100 µm or less, produced by the method according to any one of Claims 1-14.

16. Medicine, feedstuff or food comprising the microparticles according to Claim 15.
